# EUROPEAN PATENT APPLICATION

(11) **EP 4 312 019 A1**
(43) Date of publication of application: **31.01.2024**
(21) Application number: 23187729.1
(22) Date of filing: 26.07.2023
(51) Int. Cl.: G01N 21/95, G01N 21/952, G06T 11/00, G01B 11/24, A61F 2/95, A61M 25/01, A61M 25/10, G06Q 10/0631, G06Q 10/20, G06N 20/00, G01N 21/88

(54) **INSPECTION APPARATUS**

(30) Priority: 27.07.2022 US 202217874418
(71) Applicant: BIOSENSE WEBSTER (ISRAEL) LTD., 2066717 Yokneam (IL)
(72) Inventor: GOVARI, Assaf, 2066717 Yokneam (IL)
(74) Representative: Carpmaels & Ransford LLP

(57) **Abstract**

A jig for inspecting a tip of a catheter at a first facility of at least two facilities at which the catheter is processed during manufacturing. The jig includes a housing, at least one clamp, a plurality of cameras mounted in the housing, a plurality of illuminators mounted in the housing and a processor. The processor operates the plurality of cameras and the plurality of illuminators for capturing a plurality of images, and transfers respective copies of the plurality of images to a second facility, separate from the first facility. The processor is additionally configured to generated a report generate a report of the inspection from the first facility and the second facility based on annotations received from the first facility and the second facility.

## Description

### FIELD OF THE DISCLOSURE

This disclosure relates generally to inspection of medical equipment, and specifically to detecting defects in catheters.

### BACKGROUND

Before use, medical equipment should be checked to ensure there are no defects present in the equipment. Some examples of systems for inspecting medical equipment are described below:
U.S. Patent Application 2009/0251535, to Maehringer-Kunz et al., describes a device for automatic illumination and inspection of tubular probes. The device is stated to have rotatable means for holding the probes that are to be inspected, and to have an electronic camera and means for illuminating the probes that are to be inspected.
U.S. Patent 7,578,165, to Stupecky, describes measurement devices and methods for measuring balloon catheters. The devices are stated to enable measuring the outer dimensions and burst pressure of a balloon catheter.
U.S. Patent 5,234,411, to Vaillancourt, describes a container assembly that has a pierceable membrane through which the hollow needle of a connector may pass to permit pre-inflation of a balloon. The system is stated to be for use in conjunction with a flow directed type catheter for viewing the expansion of the balloon-tipped end of the flow directed type catheter within a sealed chamber of the container assembly.
U.S. Patent Application 2005/0283178, to Flagle et al., describes a delivery system that is stated to facilitate a visual inspection of an intraluminal medical device included in the delivery system.

### BRIEF DESCRIPTION OF THE DRAWINGS

Fig. 1 is a schematic diagram illustrating an inspection system for a catheter;
Fig. 2 is a schematic drawing of a jig of the system;
Fig. 3 is a flowchart listing steps performed in generating a corpus of data that is used to train an artificial neural network;
Fig. 4 is a schematic block diagram illustrating the overall structure and operation of the artificial neural network; and
Fig. 5 is a flowchart of an algorithm for training the artificial neural network.

### DESCRIPTION OF EXAMPLES

### OVERVIEW

A stage in the production of a medical catheter is inspection of the catheter tip to ensure that the tip conforms with specifications that have been defined for the catheter tip. The specifications defined are typically objective specifications, such as dimensions of elements of the tip and other properties such as impedances of electrodes of the tip. The inspection, which may be manual and/or automatic, typically generates a list of parameter values corresponding to the specifications, and review of the list determines whether or not the catheter tip is suitable or unsuitable for use.

However, the inventors have found that manual inspection of the catheter tip in practice typically generates situations where, while conforming with the objective specifications, the catheter tip may still be considered to be unsuitable for use. For example, one electrode on the tip may appear to have a matte finish, compared to other electrodes that have a reflective finish. Or, one of the electrodes may have a scratch. As a further example, in the case of a basket catheter the catheter spines may deploy asymmetrically.

Such situations are typically subjective, revealing subjective defects of the tip that are recognized in response to the manual inspection of the tip, and that may not have been included in the objective specifications. The manual inspection may be performed at a first facility, i.e., where the catheter is produced. The manual inspection may also be performed at other facilities, e.g., at a facility that receives the catheter after initial production, and that further processes the catheter. Because of the subjective nature of the manual inspection, defects identified by the two inspections may not correspond, e.g., the inspection at one of the other facilities may identify an asymmetry in a deployed balloon catheter, whereas such an asymmetry may not be registered in the first facility.

The present disclosure addresses the problems illustrated by the examples above by providing images of a catheter tip to a local inspector and to a remote inspector. The catheter is placed in a jig at the local manufacturing or production facility. The jig is pre-configured with lighting, e.g., illuminators, that illuminates the tip, and cameras to image the tip. The lighting and cameras are mounted on a plurality of different planes of the jig so that tip may be inspected from all sides. As stated above, the tip images are provided to the inspectors at different facilities, and the inspectors respectively annotate the images with identified defects and list the defects they have identified. In this manner, inspectors from different facilities may share information and together reach a decision regarding the suitability of the catheter, i.e., whether the catheter will function correctly, without the need to perform an additional inspection at the remote site. Based on the combined input from the different facilities, the local inspector may decide if the catheter passes/fails inspection and generate a report that includes the decision reached based on the combined input. The report may be stored in memory and also shared with each of the manufacturing facilities.

The process of generating images, local annotated images, remote annotated images, local lists of annotations, remote lists of annotations, and forming a decision is repeated for other catheters, all of the same type, that are placed in the jig. This generates a sub-corpus of data (raw images, local annotated images, remote annotated images, local lists of annotations, remote lists of annotations, decisions) for the type of catheter tip being inspected.

In some example embodiments, the data sub-corpus is used to train an artificial neural network to recognize subjective-type defects in the type of catheter tip used to build the sub-corpus. Once trained, the network is used to identify automatically, from raw images of a catheter under test in the jig, subjective-type defects that would typically only be found by manual inspection of the catheter tip.

Optionally, the jig may be configured for imaging different types of catheter tips. For each different type the lighting and the cameras of the jig may be adjusted manually or automatically to provide optimal images of the tips. A data sub-corpus may be assembled for each different type, and the network may be trained using the different sub-corpuses, substantially as described above, to identify automatically subjective-type defects for each of the different types. The multiple data sub-corpuses form a corpus of data, and it will be understood that by training the network with this corpus, the network is able to identify automatically subjective-type defects for all the different types.

In operation at the production facility, in some examples of the invention the jig may be configured to identify the different types of catheter tips automatically, and to adjust the lighting and the cameras accordingly. Once the type of tip has been identified, the trained network may be used to identify automatically subjective-type defects of the tip.

While the description above summarizes how a trained network may be used automatically to identify subjective-type defects of a catheter tip from images of the tip, it will be appreciated that the network may also be trained to identify objective defects in the tip from the images. Such objective defects comprise, for example, deviations from prescribed dimensions of the tip or its elements, such as the radius and/or length of a focal catheter, and/or the location and width of electrodes in focal or other catheters.

It will thus be appreciated that a trained network may be used to identify both subjective and objective defects in catheter tips, as well as to generate a recommendation as to whether the catheter should pass or fail inspection and report that recommendation to the user. The generated recommendation may include images, annotations on the images, notes from the various inspectors if received and the generated recommendations. Images prior to added annotations are referred to herein as raw images.

### SYSTEM DESCRIPTION

In the following description, like elements in the drawings are identified by like numerals, and like elements are differentiated as necessary by appending a letter to the identifying numeral.

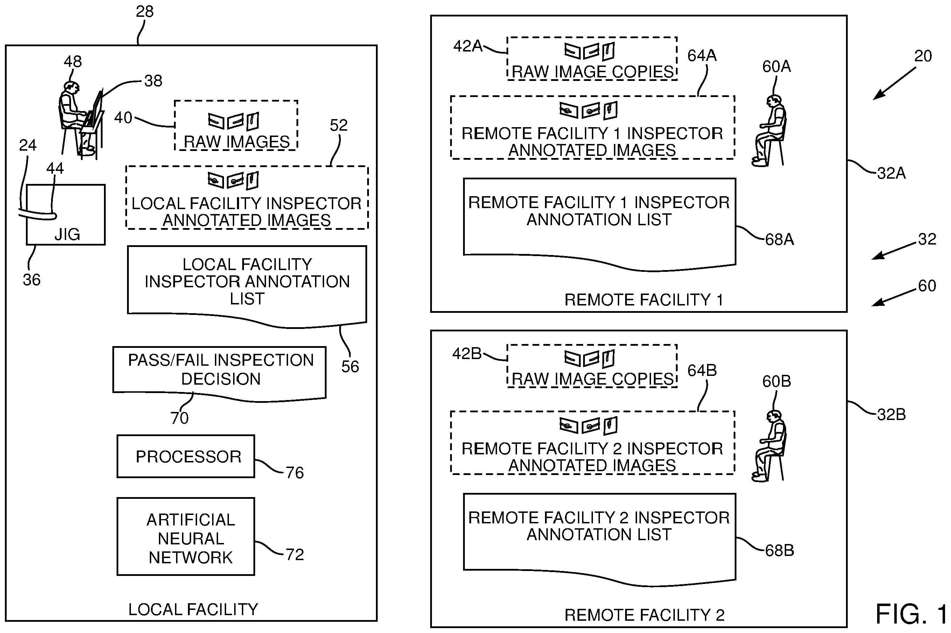

Fig. 1 is a schematic diagram illustrating an inspection system 20 for a catheter 24. System 20 is operated in a local facility 28, where the catheter is produced, and in one or more remote facilities 32A, 32B, where the catheter, or another catheter similar to catheter 24, is to be further processed. Herein local facilities 24 refers facility to at which a jig 36 is situated and remote facilities 32A, 32B are also generically termed remote facilities 32, and it will be understood that these facilities are physically separate from the local facility, and typically process the catheter by providing over additional manufacturing steps, assembly, and/or inspection operations. Local facility 28 is also termed herein production facility 28. By way of example Fig. 1 illustrates two generally similar remote facilities 32A, 32B, but in examples of the invention there may be one or more remote facilities.

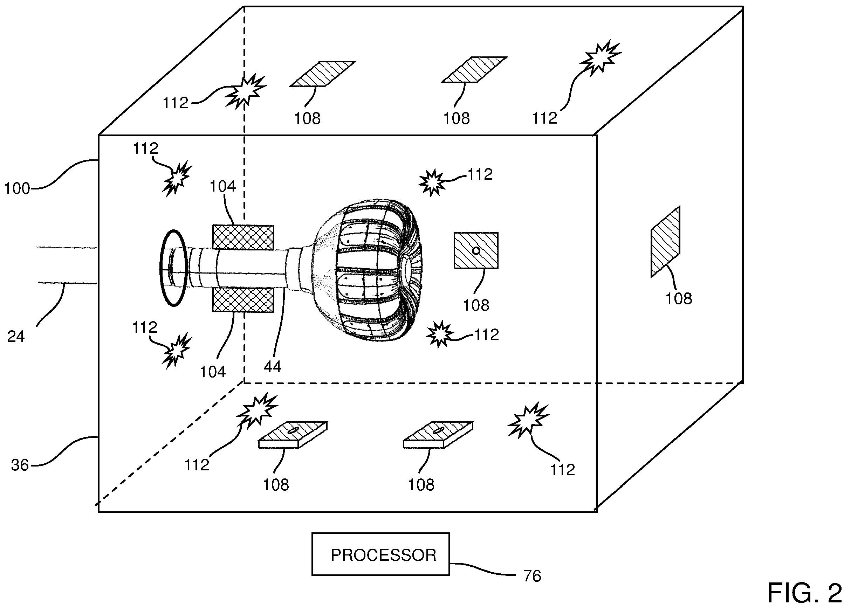

Local facility 28 comprises jig 36, described in more detail below with respect to Fig. 2, which is configured to receive catheter 24, and which is also configured to generate images 40 of a tip 44 of the received catheter. Generated images 40, herein also termed "raw" images 40, are inspected at the local facility by a local inspector 48. From the inspection the local inspector annotates the images indicating defects apparent in the images, generating a set of annotated images 52. The local inspector also generates a corresponding list of the defects, in a local facility annotation list 56.

As stated above catheter 24 is additionally processed in remote facilities 32, so that copies of raw images 40 are also transmitted to the remote facilities, as raw image copies 42A, 42B, generically termed raw mage copies 42. At remote facilities 32A, 32B respective remote inspectors 60A, 60B, generically termed inspectors 60, inspect the raw image copies. As for the local facility, at the remote facilities remote inspectors 60A, 60B annotate the raw image copies indicating defects apparent in the images, generating respective sets of remote annotated images 64A, 64B. The remote inspectors also generate respective corresponding lists of the defects, as remote facility annotation lists 68A, 68B.

Based on the inspection of both the local inspector 48 and the remote inspectors 60A, 60B a conclusion may be reached to determine if the catheter passes inspection before transporting the catheter to the customer or another remote facility. The conclusion is typically reached by local inspector 48 using a graphic user interface 38 to view the raw images, the local and remote annotated images, and the corresponding lists, so as to make a pass/fail inspection decision 70 whether the catheter is fit-for-purpose, i.e. will function correctly. The decision typically comprises generating a report optionally including images captured, annotations on the images, comments and a pass/fail recommendation or decision.

As is described further below, in some example embodiments, the raw images, local and remote annotated images, local and remote annotation lists, and pass/fail decisions as to functionality, form a data corpus that is used to train an artificial neural network (ANN) 72 that is controlled by a processor 76. Processor 76 is configured to control other elements of system 20, e.g., jig 36, and both the processor and ANN 72 are typically, but not necessarily, located at the local facility. Processor 76 may be workstation including a user interface for operating jig 36 and memory capability.

Fig 2 is a schematic drawing of jig 36. By way of example jig 36 is assumed to be constructed within a housing 100 that is a rectangular parallelepiped, and the jig is illustrated as being used on catheter 24 that is a balloon catheter. However, it will be appreciated that in examples of the invention housing 100 may be any convenient shape, and that jig 36 may be used for catheters other than balloon catheters.

Jig 36 comprises at least one clamp 104, herein by way of example assumed to comprise clamps 104 that are configured to grip tip 44 of catheter 24 while the tip is being imaged within the jig. Mounted in jig 36, typically but not necessarily in the walls of jig 36, are one or more cameras 108, and one or more illuminators 112. The multiple cameras 108 and illuminators 112 provide capturing images of tip 44 from different angles so that tip 44 can be inspected from all sides. For any given type of catheter 24, e.g., a focal catheter, a basket catheter, or a balloon catheter, clamps 104, cameras 108, and illuminators 112 may be initially adjusted manually by a jig adjuster and/or may be automatically adjusted based on identification of the catheter. In one example, and as is assumed below, the jig adjuster is local inspector 48, who is also referred to herein as adjuster 48. The manual adjustments by adjuster 48 are made so that a set of raw images 40 of the catheter, generated when the cameras and illuminators are operative, provide images of the catheter and of its tip that are acceptable in detail and in quality to the jig adjuster. While each raw image 40 may be two-dimensional, the set of raw images is selected so that the catheter and its tip are completely imaged, i.e., a complete three-dimensional view of the catheter and its tip may be derived from the set of two-dimensional images.

The clamp adjustments referred to above ensure, *inter alia,* that the no part of tip 44 is obscured in the set of raw images, and positions and orientations of clamps 104 relative to the tip and to cameras 108 and illuminators 112 are set accordingly by adjuster 48 or based on instructions generated by processor 76.

Adjuster 48 or processor 76 set parameters for each camera 108 and/or selects cameras 108 based on its operating parameters, e.g., its field of view, its focused position, wavelength and/or polarizations of radiation that generate an image in the camera. The wavelengths typically include portions of the visible and the near-visible spectrum, and in some examples one or more filters of wavelength and/or polarization may be used for the camera as it acquires an image. In some examples any given camera 108 may be configured to generate a plurality of images at different respective times, with the parameters of the camera being set differently for each image.

Adjuster 48 or processor 76 also set parameters for each illuminator 112 and or select illuminators 112 based on its operating parameters, e.g., its position and orientation, the solid angle into which its radiation is transmitted, its brightness, and the wavelengths and or polarizations of radiation emitted by the illuminator. As for the cameras, one or more filters may be used for a given illuminator 112.

In some examples, a tip 44 of a given catheter 24 may have more than one state, typically when deployed or not deployed. For example, the balloon catheter illustrated may be used in an undeployed state, with tip 44 collapsed, when the catheter tip is inserted into a patient, and may be converted to a deployed state, with tip 44 inflated, when tip 44 is in a chamber of the patient heart. The inflated and state may be implemented by applying a pressure differential between the balloon interior and jig 36. As another example, a basket catheter may be used in an undeployed state, with the basket tip collapsed, when the basket tip is inserted into the patient, and may convert to a deployed state, with the basket tip expanded, when the tip is in the patient heart. (In contrast, a focal catheter, i.e., a catheter with a focal tip, typically has one state both in a deployed or undeployed state.)

In some example embodiments, for a given catheter having tip 44 with multiple states, clamps 104, cameras 108, and illuminators 112 are adjusted for each of the states, so that images of tip 44 in each of the states are acceptable in detail and in quality to the jig adjuster. Optionally, the adjustments for each of the multiple states are sequentially activated.

Optionally, the adjustment settings referred to above, of clamps 104, cameras 108, and illuminators 112, are made using respective actuators, such as linear and/or rotary actuators. Additionally or alternatively, the adjustment settings for cameras 108 and/or the illuminators may comprise setting values of electrical parameters to operate cameras 108 and/or illuminators 112, including exposure times for cameras 108 and powered-on times for illuminators 112. For each state of a given tip 44, once the actuators have been adjusted manually, the settings for each of the actuators, and the values of the electrical parameters, may be provided to processor 76, and processor 76 may use the settings to operate jig 36 for other tips 44 of catheters similar to catheter 24.

In some examples of the invention, processor 76 may use initial images acquired by one or more cameras 108 to identify which catheter 24 and which tip 44 has been introduced into jig 36. In some examples the initial images are acquired before clamps 104 are actuated, so that tip 44 is placed in jig 36 without being gripped by the clamps. Once identified, processor 76 may activate the clamps, cameras 108, and illuminators 112, so that after tip 44 has been fixedly clamped images 40, subsequent to the initial images, may be acquired automatically. Alternatively, adjuster 48 may provide the identification of catheter 24 and tip 44 to processor 76, which may then activate the clamps, cameras, and illuminators according to the settings previously provided to the processor, as described above.

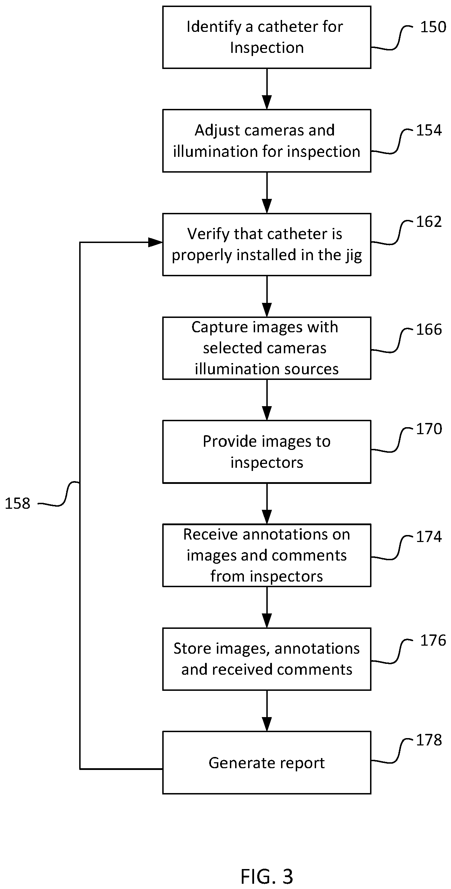

Fig. 3 is a flowchart 152 listing steps performed by jig 36 for generating a report summarizing inspection performed by a plurality of inspectors at different facilities. In some example embodiments, data collected and stored by jig 36 is used in generating a corpus of data for training artificial neural network 72. The data corpus is typically for a plurality of different tips 44 of catheters 24, so that the steps of the flowchart are followed for each different tip 44. I.e., for each different tip a respective sub-corpus of data is generated. The description of the steps of the flowchart is assumed to be for system 20, i.e., one local facility and two remote facilities, and those having ordinary skill in the art will be able to modify the description for numbers of remote facilities different from two.

In an initial setup step 150 a catheter for inspection is identified by jig 36. Optionally, local inspector 48 selects a catheter 24 including tip 44 to be tested, herein also termed the target tip and communicates that selection to jig 36 via a user interface of jig 36. In a jig setup step 154, cameras 108, and illuminators 112 are configured for capturing images of the tip. Optionally, the configurations to be used for the selected catheter are stored in memory of jig 36. Optionally, the local inspector introduces tip 44 into jig 36, and adjusts elements of jig 36, i.e., clamps 104, cameras 108, and illuminators 112. Optionally, the images of the tip acquired by cameras 108 provide inspecting tip 44, as described above. Where applicable, the elements of jig 36 are adjusted so that images are acquired for each state of the catheter tip. The settings used to generate the images are stored for use by processor 76 in the remaining steps of the flowchart.

In a first operative step 162, proper installation of the catheter in the jig may be verified. Optionally, local inspector 48 inserts tip 44 of the catheter type for which jig 36 has been setup, in steps 150 and 154. Once jig 44 has identified tip 44, processor 76 adjusts clamps 104, cameras 108, and illuminators 112 according to the settings stored for tip 44.

In an imaging step 166, processor 76 operates cameras 108 and illuminators 112 to generate and acquire raw images 40 of tip 44.

In an image provision step 170 raw images 40 are provided to local inspector 48, and copies of the raw images 42A, 42B are also provided to remote inspectors 60A, 60B in facilities 32A, 32B. While typically the raw images and their copies are provided electronically, in some cases at least some of the raw images may be provided as "hard copy," i.e., on paper. The local inspector then annotates raw images 40 and lists the annotations made as local facility inspector annotated images 52 and local facility inspector annotation list 56. Raw images that are annotated are referred to herein as annotated images. Raw images as referred to herein are images prior to annotation. Similarly, the remote inspectors respectively annotate raw image copies 42A, 42B and list the annotations made as remote facility inspector annotated images 62A, 62B and remote facility inspector annotation lists 68A, 68B.

In a data collation step 174, processor 76 receives and stores the raw images and the annotated images, as well as the respective lists of the annotations, as a set of data for the catheter being tested.

In an inspection step 176, processor 76 provides, to user interface 38, the set of data for the catheter being tested, i.e., the raw images, the annotated images, and the annotation lists. Local inspector 48 may display the data on the user interface to decide whether the catheter is fit-for-purpose, i.e., whether it will function correctly. Processor 76 stores the pass/fail decision 70 for the catheter, and applies it as a pass/fail inspection notification to the catheter being tested.

In a reporting step 178, jig 36 generates a report including one or more of the captured raw images, comments and a pass/fail decision. The report may be stored in memory associated with processor 76 and optionally shared over the cloud with the remote sites.

The operating steps may be repeated as illustrated by arrow 158, for other catheters of the same type as identified in initial step 150, each iteration providing a set of data and a decision, the multiple sets and decisions forming a sub-corpus of data for the catheter being tested.

The steps of the flowchart of Fig. 3 are typically repeated for other catheter types, each catheter type generating a respective sub-corpus of data. The multiplicity of sub-corpuses forms a corpus of data that is used to train artificial neural network (ANN) 72.

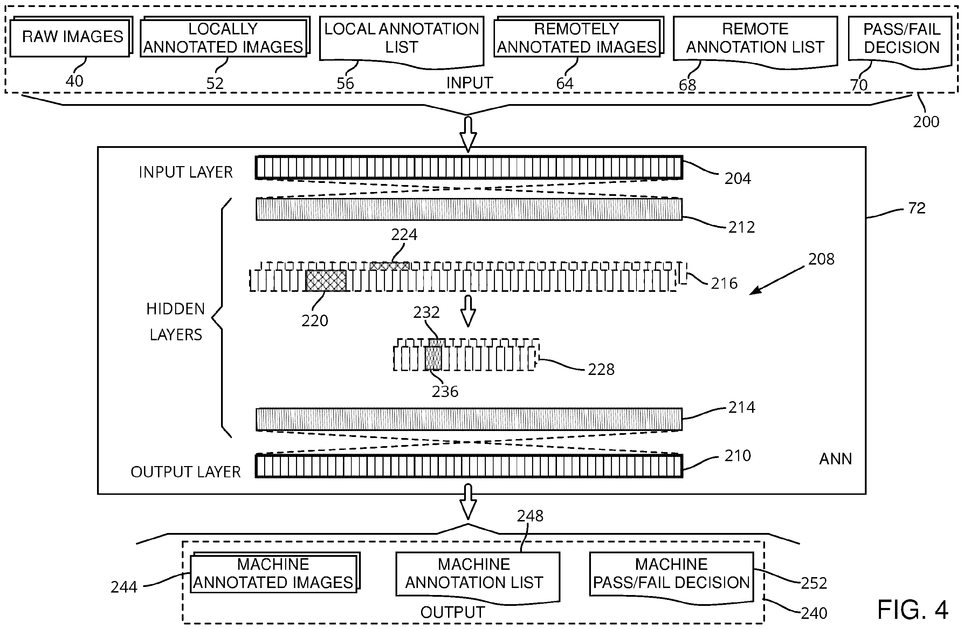

Fig. 4 is a schematic block diagram illustrating the overall structure and operation of ANN 72. It will be understood that the structure and operation presented here are by way of example, and those having ordinary skill in the art will be aware of networks, having other structures and operations, that function in a similar manner to ANN 72. All such networks are assumed to be comprised within the scope of the present invention.

In the illustrated figure, ANN 72, when training for a selected tip 44 of a selected catheter 24, is configured to receive, as an input signal 200, the relevant sub-corpus of data stored in step 174 of flowchart 152. I.e., input signal 200 comprises raw images 40, local facility inspector annotated images 52, local facility inspector annotation list 56, remote facility inspector annotated images 64, remote facility inspector annotation list 68, and pass/fail inspection decision 70. An output signal 240 of the network is described further below.

ANN 72 is formed of layers of artificial neurons, and hereinbelow each of the layers is assumed to comprise rectified linear unit (ReLU) neurons. However, the layers of ANN 72 may be comprised of other neurons, such as derivations of ReLU neurons, tanh neurons and/or sigmoid neurons, and those having ordinary skill in the art will be able to adapt the disclosure, *mutatis mutandis,* for layers with other such neurons.

ANN 72 has a first input layer 204, which is followed by a number of hidden layers 208, and the hidden layers are followed by an output layer 210. These layers are described in more detail below.

In a disclosed example of ANN 200, input layer 204 has a number of neurons corresponding to the number of data elements in input 200. The data elements of any given input 200 comprise pixels of raw image 40 and of annotated images 52, 64, and typically the data elements of the annotated image only comprise the pixels of the defects. The data elements of the input also comprise indicators of the listed defects in lists 56, 68. Typically the lists of the defects are indexed, so that there is a one-one correspondence between an alphanumeric index, acting as a defect indicator, and a description of a specific defect.

Hidden layers 208 are formed as a plurality of parallel sets of layers each set typically comprising at least one convolution layer and/or one fully connected layer. ANN 72 is illustrated as having two fully connected layers 212, 214, with layer 212 following input layer 204 and layer 212 preceding output layer 210. One convolutional layer 216 is shown in Fig. 4. It will be appreciated that the depiction of ANN 72 as having two fully connected layers and one convolutional layer is purely illustrative, and that in practice ANN 72 may have a plurality of fully connected layers, substantially similar to layer 212 and/or a plurality of convolutional layers substantially similar to layer 216.

In the illustrated example convolution layer 216, which consists of at least one filter, or kernel, is configured to perform the convolution of the layer by scanning across the values derived from input layer 204 or from another previous hidden layer. Each kernel is a filter, and the illustrated example depicts layer 216 comprising a first kernel 220 and a second kernel 224. Also, while the illustration shows that the convolution layer has two kernels, there are typically more than two kernels.

Kernels in a convolution layer, such as in layer 216, are typically configured, by their convolution operation, to filter or isolate a feature of the data being analyzed. The kernels operate by sliding, in a step manner with a preset stride, along a presented set of data, and forming convolutions of the section of data "covered" by the kernel after each step. Typically padding elements are added to the initial and terminal elements of the convolution layers to ensure that the convolution of these elements by the kernels is correct.

Convolution layer 216 is typically followed by a pooling layer 228 which is configured to receive the output of layer 216. Pooling layer 228 in turn comprises at least one filter, associated with the layer, that is configured to reduce the data generated by each of the kernels of convolution layer 216. By way of example, pooling layer 228 is assumed to have two filters 232, 236. The filters of the pooling layer do not perform convolution, but rather are configured to reduce the data from layer 216, for example by taking the maximum value of the filtered data, in which case pooling layer 228 is a max-pooling layer.

As stated above, the depiction of ANN 72 is illustrative, and typically there is a multiplicity of convolutional layers similar to layer 216, each convolutional layer being followed by a respective pooling layer.

In an illustrated disclosed example, output layer 210 is preceded by a fully connected layer 214, the sizes of the layers typically being selected to correspond to the size of the data output by ANN 72. ANN 72 is trained so that its data output signal 240 comprises machine annotated images 244, a machine annotation list 248, and a machine pass/fail decision 252. Machine annotated images 244 are generally similar to the local and remote annotation images, comprising sets of pixels, each set illustrating a defect in the catheter tip being inspected, where the sets of pixels are selected by ANN 72. Machine annotation list 248 is generally similar to lists 56, 68, and typically comprises alphanumeric indices, selected by ANN 72, that have a one-one correspondence with the sets of pixels in images 244. Machine pass/fail decision 252 is generally similar to pass/fail inspection decision 70, typically comprising an index corresponding to pass or fail for the catheter being inspected.

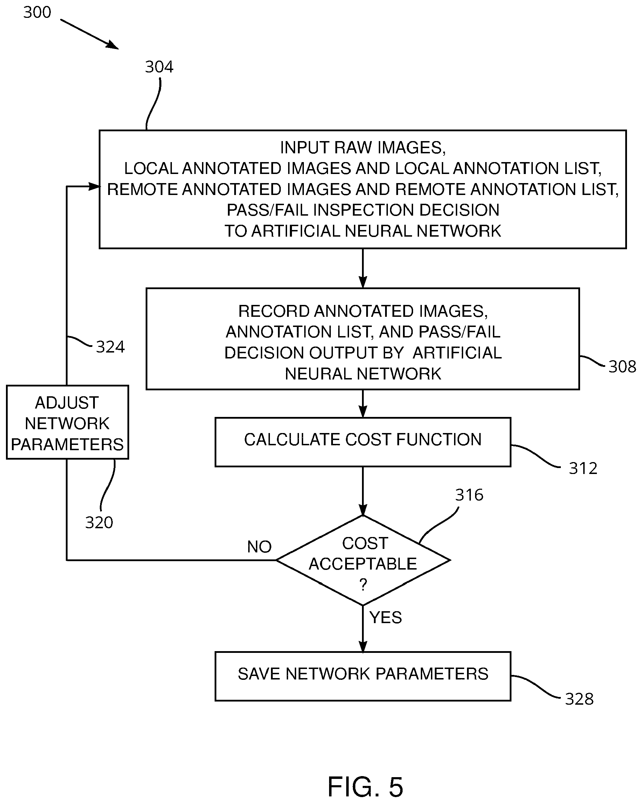

Fig. 5 is a flowchart 300 of an algorithm for training ANN 72. The training is an iterative process, wherein parameters of the network, such as the weights of the network neurons, the numbers and sizes and weights of the filters of the convolutional layers, and the number and type of the different layers, are adjusted so as to optimize the output of the network. The training is assumed to be performed using processor 76, but any other suitable processor may be used.

In an initial step 304 a sub-corpus of data, that has typically been produced using the algorithm of Fig. 3, is input to input layer 204. Because of the large number of signals that may be in the sub-corpus, the input of the signals is typically implemented as a batch process.

In an evaluation step 308, the processor records the annotated images, the annotation list, and the pass/fail decision calculated and output by the network, and in a cost calculation step 312 the processor, using the known annotation images, lists, and decisions of the input batch of signals, calculates a cost relating the known annotated images, lists, and decisions to the annotated images, the list, and the decision output by the network.

Step 312 may use any cost function known in the art, such as a quadratic cost function or a cross-entropy cost function.

In a decision step 316 the processor determines if the cost calculated in step 312 is low enough, so as to be acceptable. If the cost is too high, i.e., the cost is not acceptable so that the decision returns negative, the processor, in an adjustment step 320, alters the parameters of the network using any suitable optimization algorithm, for example, a gradient descent algorithm such as the Adam optimizer.

As stated above, and as illustrated by arrow 324, the training process is an iterative process, so that from step 324 the processor returns to initial step 304.

If decision step 316 returns positive, i.e., the cost calculated in step 312 is sufficiently low, then in a save step 328 the processor saves the network parameters that have been calculated, and uses these in ANN 72.

The description above of flowchart 300 illustrates how ANN 72 may be trained for a sub-corpus of data corresponding to a given catheter type. The steps of the flowchart may be repeated for all sub-corpuses, corresponding to all catheter types produced in local facility 28, so as to train ANN 72 to detect defects in all catheter types produced in the local facility.

### EXAMPLES

Example 1. A method, comprising:
receiving images (40), of a catheter (24) configured to be inserted into a lumen of a human subject, captured in a production facility (28) of the catheter;
receiving first annotations (52) on the images of first defects of the catheter;
formulating, at a further facility (32A), separate from the production facility, processing the catheter, second annotations (64A) on respective copies (42A) of the images of second defects of the catheter; and
training an artificial neural network (72) using the images, the first annotations, and the second annotations to detect defects in further catheters produced in the production facility.

Example 2. The method according to example 1, and comprising providing a jig (36) at the production facility that is configured to capture the images.

Example 3. The method according to example 2, wherein the images comprise initial images, and wherein the jig is configured to identify a type of the catheter from the initial images.

Example 4. The method according to example 3, wherein the images used to train the artificial neural network comprise images acquired subsequent to the initial images.

Example 5. The method according to example 3, wherein the further catheters produced in the production facility have the identified type of the catheter.

Example 6. The method according to example 2, wherein the jig is configured to receive and fixedly grip the catheter prior to capturing the images.

Example 7. The method according to example 6, wherein the images comprise images of a tip (44) of the catheter, the method further comprising adjusting at least one clamp (104) of the jig to fixedly grip the catheter so that no part of the tip is obscured in the images of the tip.

Example 8. The method according to example 1, wherein the catheter comprises a tip (44), and wherein the received images comprise tip images, and wherein training the artificial network comprises training the network, using the tip images, first annotations of the tip images, and second annotations of respective copies of the tip images, to detect defects in tips of the further catheters.

Example 9. The method according to example 8, wherein the tip of the catheter has a plurality of states, and wherein training the artificial network comprises training the network, to detect defects in tips of the further catheters in the plurality of states.

Example 10. The method according to example 9, wherein a first state of the plurality comprises the tip in an undeployed state corresponding to the tip being within the lumen, and a second state comprises the tip in a deployed state corresponding to the tip being in a heart of the human subject.

Example 11. The method according to example 1, and comprising formulating a pass/fail inspection decision (70) for the catheter in response to the images, the first annotations, and the second annotations, and training the artificial neural network using the decision to formulate further pass/fail inspection decisions for the further catheters produced in the production facility.

Example 12. Apparatus, comprising:
an artificial neural network (72); and
a processor (76), configured to
receive images (40), of a catheter (24) configured to be inserted into a lumen of a human subject, captured in a production facility (28) of the catheter,
receive first annotations (52) on the images of first defects of the catheter,
receive second annotations (64A), on respective copies (42A) of the images of second defects of the catheter, formulated at a further facility (32A), separate from the production facility, processing the catheter, and
training the artificial neural network using the images, the first annotations, and the second annotations to detect defects in further catheters produced in the production facility.

Example 13. The apparatus according to example 12, and comprising a jig (36) located at the production facility that is configured to capture the images.

Example 14. The apparatus according to example 13, wherein the images comprise initial images, and wherein the jig is configured to identify a type of the catheter from the initial images.

Example 15. The apparatus according to example 14, wherein the images used to train the artificial neural network comprise images acquired subsequent to the initial images.

Example 16. The apparatus according to example 14, wherein the further catheters produced in the production facility have the identified type of the catheter.

Example 17. The apparatus according to example 13, wherein the jig is configured to receive and fixedly grip the catheter prior to capturing the images.

Example 18. The apparatus according to example 17, wherein the images comprise images of a tip (44) of the catheter, and comprising at least one clamp (104) of the jig that fixedly grips the catheter and that is adjusted so that no part of the tip is obscured in the images of the tip.

Example 19. The apparatus according to example 12, wherein the catheter comprises a tip (44), and wherein the received images comprise tip images, and wherein training the artificial network comprises training the network, using the tip images, first annotations of the tip images, and second annotations of respective copies of the tip images, to detect defects in tips of the further catheters.

Example 20. The apparatus according to example 19, wherein the tip of the catheter has a plurality of states, and wherein training the artificial network comprises training the network to detect defects in tips of the further catheters in the plurality of states.

Example 21. The apparatus according to example 20, wherein a first state of the plurality comprises the tip in an undeployed state corresponding to the tip being within the lumen, and a second state comprises the tip in a deployed state corresponding to the tip being in a heart of the human subject.

Example 22. The apparatus according to example 12, and comprising formulating a pass/fail inspection decision (70) for the catheter in response to the images, the first annotations, and the second annotations, and training the artificial neural network using the decision to formulate further pass/fail inspection decisions for the further catheters produced in the production facility.

Example 23. A jig (36) for inspecting a tip (44) of a catheter (24) at a first facility (52) of at least two facilities (52,64) at which the catheter (24) is processed during manufacturing, the jig (36) comprising ;a housing (100) configured to house the tip (44) of the catheter (24) during inspection; at least one clamp (104) configured to fixedly position the tip (44) within the housing (100); a plurality of cameras (108) mounted in the housing (100), said plurality of cameras (108) configured to capture images (40) of the tip (44) from different angles; a plurality of illuminators (112) mounted in the housing, said plurality of illuminators (112) configured to illuminate the tip (44) from different angles; a processor (76), configured to: selectively operate the plurality of cameras (108) and the plurality of illuminators (112) based on the catheter (24) mounted within the housing (100), transfer respective copies of the plurality of images (40) to a second facility (68), separate from the first facility (56), processing the catheter (24), receive first annotations on the images of defects of the catheter (24) from the first facility (56), and receive second annotations on the respective copies of the images of defects of the catheter (24) from the second facility (68); and generate a report of the inspection from the first facility (56) and the second facility (68) based on the first annotations and the second annotations; wherein the processor (76) is associated with a user interface for interfacing with a user and memory for storing the plurality of images include the first annotations and second annotations (40, 52, 56).

Example 24. The apparatus according to example 23, wherein the images comprise initial images, and wherein the processor is configured to identify a type of the catheter from the initial images.

Example 25. The jig according to example 23, wherein the plurality of cameras (108) includes at least two cameras mounted on different walls of the housing (100) .

Example 26. The jig (36) according to example 23, wherein the plurality of cameras (108) includes at least one camera that captures images at a wavelength or polarization that is other than that of another camera from the plurality of cameras (108).

Example 27. The jig (36) according to example 23, wherein the plurality of illuminator includes at least one illuminator that emits light at a different wavelength than that of another illuminator from the plurality of illuminators.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A jig (36) for inspecting a tip (44) of a catheter (24) at a first facility (52) of at least two facilities (52,64) at which the catheter (24) is processed during manufacturing, the jig (36) comprising:
a housing (100) configured to house a tip (44) of the catheter (24) during inspection;
at least one clamp (104) configured to fixedly position the tip (44) within the housing (100);
a plurality of cameras (108) mounted in the housing (100), said plurality of cameras (108) configured to capture images (40) of the tip (44) from different angles;
a plurality of illuminators (112) mounted in the housing, said plurality of cameras configured to illuminate the tip (44) from different angles;
a processor (76), configured to:
selectively operate the plurality of cameras (108) and the plurality of illuminators (112) based on the catheter (24) mounted within the housing (100),
transfer respective copies of the plurality of images (40) to a second facility (68), separate from the first facility (56), processing the catheter (24),
receive first annotations on the images of defects of the catheter (24) from the first facility (56), and
receive second annotations on the respective copies of the images of defects of the catheter (24) from the second facility (68); and
generate a report of the inspection from the first facility (56) and the second facility (68) based on the first annotations and the second annotations;
wherein the processor (76) is associated with a user interface for interfacing with a user and memory for storing the plurality of images include the first annotations and second annotations (40, 52, 56).

2. The jig according to claim 1, wherein the plurality of images comprise initial images, and wherein the processor is configured to identify a type of the catheter from the initial images.

3. The jig according to claim 1, wherein the plurality of cameras (108) includes at least two cameras mounted on different walls of the housing (100).

4. The jig (36) according to claim 1, wherein the plurality of cameras (108) includes at least one camera that captures images at a wavelength or polarization that is other than that of another camera from the plurality of cameras (108).

5. The jig (36) according to claim 1, wherein the plurality of illuminator includes at least one illuminator that emits light at a different wavelength than that of another illuminator from the plurality of illuminators.

6. Apparatus, comprising:
an artificial neural network (72); and
a processor (76), configured to
receive images (40), of a catheter (24) configured to be inserted into a lumen of a human subject, captured in a production facility (28) of the catheter,
receive first annotations (52) on the images of first defects of the catheter,
receive second annotations (64A), on respective copies (42A) of the images of second defects of the catheter, formulated at a further facility (32A), separate from the production facility, processing the catheter, and
training the artificial neural network using the images, the first annotations, and the second annotations to detect defects in further catheters produced in the production facility.

7. The apparatus according to claim 6, and comprising a jig (36) located at the production facility that is configured to capture the images, wherein the images comprise initial images, and wherein the jig is configured to identify a type of the catheter from the initial images.

8. The apparatus according to claim 7, wherein the images used to train the artificial neural network comprise images acquired subsequent to the initial images.

9. The apparatus according to claim 7, wherein the further catheters produced in the production facility have the identified type of the catheter.

10. The apparatus according to claim 7, wherein the jig is configured to receive and fixedly grip the catheter prior to capturing the images.

11. The apparatus according to claim 10, wherein the images comprise images of a tip (44) of the catheter, and comprising at least one clamp (104) of the jig that fixedly grips the catheter and that is adjusted so that no part of the tip is obscured in the images of the tip.

12. The apparatus according to claim 6, wherein the catheter comprises a tip (44), and wherein the received images comprise tip images, and wherein training the artificial network comprises training the network, using the tip images, first annotations of the tip images, and second annotations of respective copies of the tip images, to detect defects in tips of the further catheters.

13. The apparatus according to claim 12, wherein the tip of the catheter has a plurality of states, and wherein training the artificial network comprises training the network to detect defects in tips of the further catheters in the plurality of states.

14. The apparatus according to claim 13, wherein a first state of the plurality comprises the tip in an undeployed state corresponding to the tip being within the lumen, and a second state comprises the tip in a deployed state corresponding to the tip being in a heart of the human subject.

15. The apparatus according to claim 6, and comprising formulating a pass/fail inspection decision (70) for the catheter in response to the images, the first annotations, and the second annotations, and training the artificial neural network using the decision to formulate further pass/fail inspection decisions for the further catheters produced in the production facility.
